# EUROPEAN PATENT APPLICATION

(11) **EP 2 570 084 A1**
(43) Date of publication of application: **20.03.2013**
(21) Application number: 12184318.9
(22) Date of filing: 13.09.2012
(51) Int. Cl.: A61B 17/06, B29C 65/08

(54) **Looped suture**

(30) Priority: 13.09.2011 US 201161534040 P; 06.08.2012 US 201213567518
(71) Applicant: Covidien LP, Mansfield, MA 02048 (US)
(72) Inventor: Gould, Jessica, Bethany, CT Connecticut 06524 (US); Belcheva, Nadya, Hamden, CT Connecticut 06518 (US); Aminuddin, Norman, Madison, CT Connecticut 06443 (US)
(74) Representative: Maschio, Antonio

(57) **Abstract**

A method of forming a loop in a thread is provided. The method includes the steps of providing a loop forming system including, a base for securely retaining a thread to be formed and a welding assembly for forming a loop in the thread, contacting at least a portion of the thread with a super-critical fluid and securing the previously immersed portion of the thread to the base such that a first section of the previously immersed portion is maintained adjacent to a second section of the previously immersed portion. The method further includes the steps of approximating the welding assembly and the base towards each other, and approximating the welding assembly and base away from each other. Also provided is a medical device formed from the method.

## Description

### CROSS-REFERENCE TO RELATED APPLICATION

The present application claims benefit to and priority from U.S. Provisional Application Serial No. 61/534,040, filed September 13, 2011, the entire content of which is incorporated herein by reference.

### BACKGROUND

### Technical Field

The present disclosure relates to a suture having a loop. More particularly, the present disclosure relates to sections of a looped suture having contacted super-critical fluid, reinforcing the loop.

### Background of Related Art

Sutures including loops formed therein are known. A loop formed in a suture during manufacture may be used to secure the suture to tissue. In this manner, once the non-looped end of the suture is inserted through tissue, that end may be threaded through the loop to form a slip knot-like configuration that may be tied to secure tissue. In another application, a loop may be formed in a suture in place of a knot. This requires the use of a handheld instrument that may be brought into an operating room.

Regardless of the reason for forming the loop, when a loop is formed in a suture, whether using adhesive, heat or ultrasonic energy, the loop may be under an inherent stress originating from the hairpin turn the suture makes in forming the loop. In the event that the suture loop is used to secure tissue, the holding power of the loop may be placed under increased stress while the tissue is healing. This increased stress applied to the loop may result in separation or the pulling apart of the loop.

Therefore, it would be beneficial to have a looped suture of enhanced loop stability.

### SUMMARY

A method of forming a loop in a thread is provided. The method includes the steps of providing a loop forming system including, a base for securely retaining a thread to be formed and a welding assembly for forming a loop in the thread, contacting at least a portion of the thread with a super-critical fluid and securing the previously immersed portion of the thread to the base such that a first section of the previously immersed portion is maintained adjacent to a second section of the previously immersed portion. The method further includes the steps of approximating the welding assembly and the base towards each other, and approximating the welding assembly and base away from each other.

In one embodiment, the loop forming system may further include a cutting assembly for forming a taper on an end of the loop. As such, the method may further include the steps of approximating the cutting assembly and the base towards each other, cutting a tapered end on a proximal end of the first section of the thread, and approximating the cutting assembly and base away from each other. The method may also include the step of removing the formed suture from the base. The loop forming system may also include a clamping device for receiving the first end of a thread and/or a tensioning device for tensioning the thread once the thread is retained in the base. In some embodiment, the step of contacting at least a portion of the thread with a super critical fluid includes submersing the at least a portion of the thread in the super critical fluid. In other embodiments, the step of contacting at least a portion of the thread with a super critical fluid includes applying the super critical fluid to the at least a portion of the thread. The super critical fluid may be super-critical carbon dioxide (CO₂).

Another method of forming a loop in a suture is also provided. The method includes the steps of providing a length of thread having a first section and a second section, contacting at least the first section and the second section of the thread with a super-critical fluid, overlapping the first and second sections of thread, and pressing together the first and second sections of thread. The step of contacting at least first and second sections of thread with a super critical fluid may include submersing the at least first and second sections of thread in the super critical fluid. Alternatively, the step of contacting the at least first and second sections of thread with a super critical fluid includes applying the super critical fluid to the at least first and second sections of thread. The method may further include the step of cutting a tapered end on the proximal end of the first section of thread. In some embodiments, the super critical fluid is super-critical carbon dioxide (CO₂).

An additional method of forming a loop in a thread is provided. The method includes the steps of providing a loop forming system including, a base for securely retaining a thread to be formed and a welding assembly for forming a loop in the thread, providing a chamber filled with super critical fluid, securing the thread to the base such that a first section of the thread is maintained adjacent to a second section of the thread, positioning the base in the chamber, approximating the welding assembly and the base towards each other to ultrasonically weld the first and second section of thread together, wherein the, and approximating the welding assembly and base away from each other. The super critical fluid may be super-critical carbon dioxide (CO₂).

Also provided is a medical device formed from any one of the disclosed methods.

### BRIEF DESCRIPTION OF THE DRAWINGS

The accompanying drawings, which are incorporated in and constitute a part of this specification, illustrate embodiments of the disclosure and, together with a general description of the disclosure given above, and the detailed description of the embodiment(s) given below, serve to explain the principles of the disclosure, wherein:

FIG. 1A is a side view of a looped suture including a tapered portion;

FIG. 1B is a cross-sectional end view of the looped suture of FIG. 1A, taken along line 1B-1B;

FIG. 1C is an enlarged side view of portion 1C of FIG. 1A;

FIG. 2A is a front view of a base used in the tapered loop forming method of the present disclosure;

FIG. 2B is a side view of the base of FIG. 2A;

FIG. 2C is a top view of the base of FIGS. 2A and 2B;

FIG. 3A is a side view of the base of FIGS. 2A-2C loaded with a suture and including a welding assembly, a suture retaining assembly and a suture tensioning assembly;

FIG. 3B is a top view of the loaded base of FIG. 3A;

FIG. 3C is a cross-section front view of the loaded base of FIGS. 3A and 3B;

FIGS. 4A and 4B are enlarged views of FIG. 3C, with the suture in a pre-welded (FIG. 4A) and post-welded (FIG. 4B) configuration;

FIG. 5A is a top view of the loaded base of FIGS. 3A-3C, post-welding and prior to the tapered cut being formed;

FIG. 5B is a cross-sectional front view of the loaded base of FIG. 5A;

FIG. 5C is a cross-sectional side view of the loaded base of FIGS. 5A and 5B;

FIG. 6A is a cross-sectional front view of the loaded base of FIGS. 5A-5C, post-welding and post-cutting of the suture;

FIG. 6B is a cross-sectional side view of the loaded base of FIG. 6A.

### DETAILED DESCRIPTION

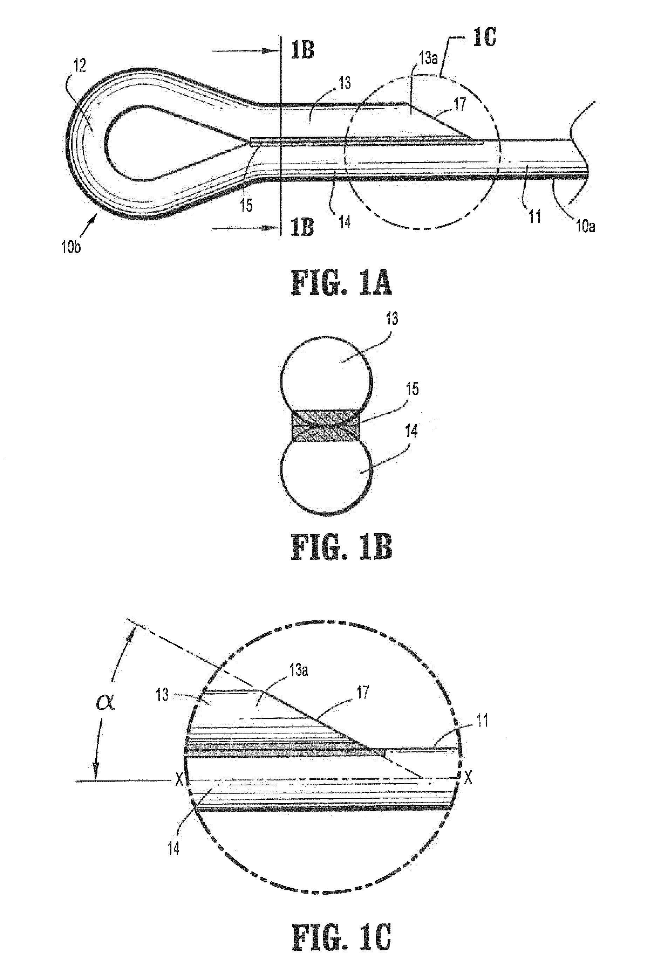

A method for increasing the strength of a looped suture including a tapered surface is herein described. Referring initially to FIG. 1A, a looped suture formed in accordance with the present disclosure is shown generally as looped suture 10. Suture 10 is formed from a monofilament thread 11; however, it is envisioned that suture 10 may be formed of braided threads, multifilament threads and other surgical fibers. Although shown having a circular cross-sectional geometry, the cross-sectional geometry of thread 11 may be of any suitable shape, such as, round, elliptical, square, flat, octagonal, and rectangular. Thread 11 may be formed of degradable materials, non-degradable materials, and combinations thereof. Thread 11 may be formed using any technique within the purview of those skilled in the art, such as, for example, extrusion, molding and/or gel spinning.

With reference to FIGS. 1A and 1B, looped suture 10 includes a loop 12 formed on a distal end 10b thereof. Loop 12 forms a substantially teardrop shape and may be formed of any size. A first section 13 of monofilament thread 11 overlays a second section 14 of thread 11 to form loop 12. The adjacent surfaces of first and second sections 13, 14 form a joined segment or joint 15. As shown, joined segment 15 extends beyond first section 13 of thread 11. In this manner, first and second sections 13, 14 of thread 11 are less likely to separate or peel away from each other as looped suture 10 is pulled through tissue (not shown).

As will be described in further detail below, first and second sections 13, 14 of thread 11 are welded together to form joined section 15. Energy is locally applied to first and second sections 13, 14 of thread 11 thereby fusing sections 13, 14 together to form joined segment 15. Various types of energy may be applied to first and second sections 13, 14 to form joined segment 15, including, radio frequency (RF), ultrasonic, laser, electrical arc discharge, and thermal. Alternatively, first and second sections 13, 14 of thread 11 may be joined using glues, epoxies, solvents, or other adhesives.

With particular reference to FIG. 1C, a proximal end 13a of first section 13 is angled to form a tapered surface 17. Tapered surface 17 angles downwardly towards proximal end 10a of looped suture 10. Tapered surface 17 may form an angle α relative to a longitudinal axis "X" of second section 14, between zero degrees (0°) and ninety degrees (90°), and preferably between about five degrees (5°) to about thirty degrees (60°). Tapered surface 17 facilitates insertion of loop 12 into or through tissue. Tapered surface 17 may be formed prior to, during or following the joining of first and second sections 13, 14. In one embodiment, tapered surface 17 is formed such that joined segment 15 extends beyond first section 13 of thread 11. In this manner, tapered surface 17 forms a smooth transition with second section 14 of thread 11, thereby decreasing the likelihood that first and second sections 13, 14 might separate or peel away from each other as looped suture 10 is pulled through tissue.

Although shown having a substantially planar taper, tapered surface 17 may include any number of configurations. For example, tapered surface 17 may be beveled, may include a laterally and longitudinally concave taper, may include a laterally and longitudinally convex taper, or may include any combination thereof. Tapered surface 17 may be selected depending on the tissue being sutured and/or the depth loop 12 is desired to be received within the tissue.

In one embodiment, prior to welding the loop, thread 11 may be soaked, immersed, or submerged in super-critical carbon dioxide (CO₂). As used herein, the term "super-critical fluid" may be used interchangeably with "densified fluid" and refers to any composition that is above a temperature and pressure at which the phase boundary (e.g., between liquid, gas, or solid) do not exist, i.e., critical state.

The thread may be soaked in super-critical CO₂ and allowed to swell. Once the material becomes softened, the thread is placed into the loop forming system described hereinbelow.

In another alternate embodiment, the first and second sections of the thread may be contacted with super-critical CO₂. As the CO₂ penetrates the thread, sections of the thread may swell and soften. The first and second sections can then be "welded" together without the application of ultrasonic energy as the polymers have softened. For example, once softened, the thread may be pressed together. The resultant joined segment may exhibit an increase in pull apart strength.

In alternate embodiments, the loop is formed on the thread in accordance with the methods described hereinbelow; however, the ultrasonic welding is performed in a chamber filled with super-critical CO₂.

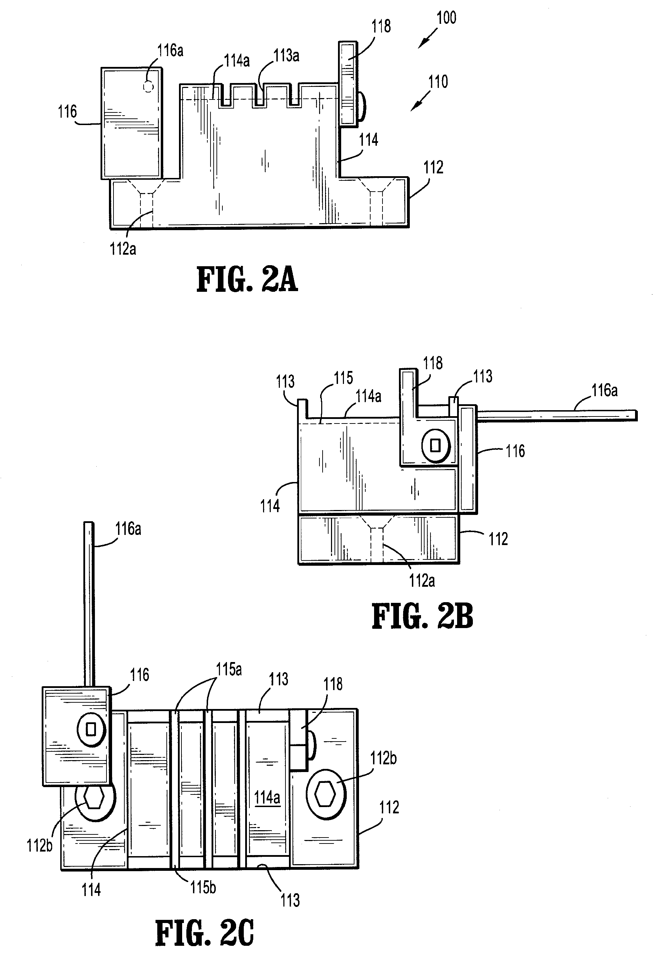

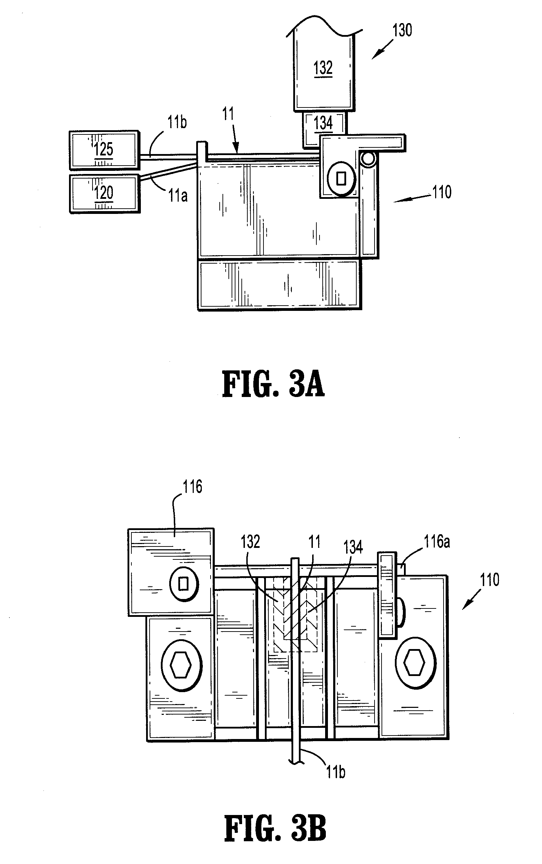

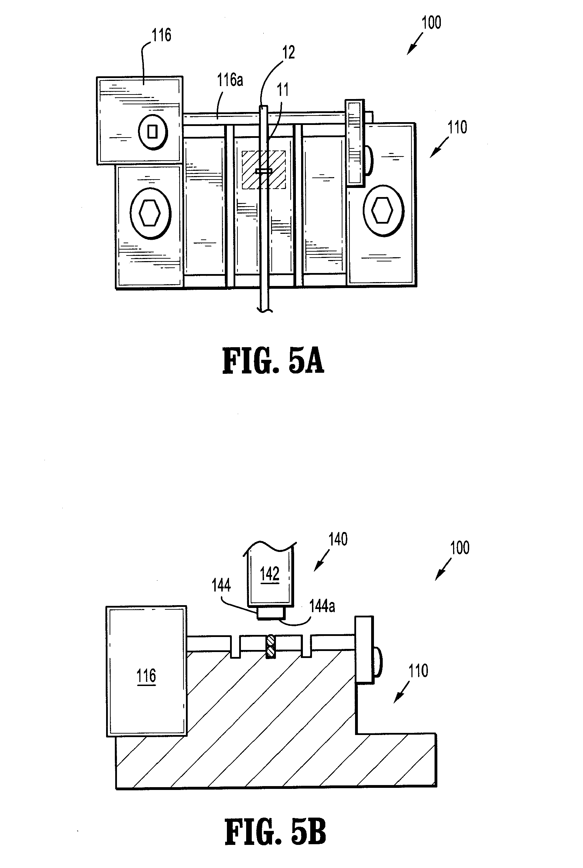

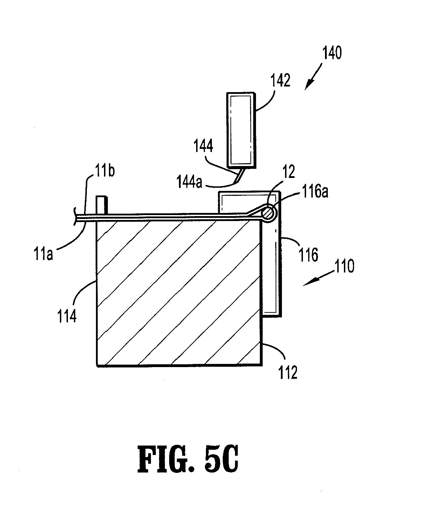

A system for forming loop 12 on distal end 10b of looped suture 10 will now be described with reference to FIGS. 2A-6B, and is shown generally as system 100. System 100 includes a fixture member or base 110, a suture retaining member 120 (FIG. 3A), a suture tensioning member 125 (FIG. 3A), a welding assembly 130 (FIGS 3A-3C), and a cutting assembly 140 (FIGS. 5A-6B).

Referring initially to FIGS. 2A-2C, base 110 includes a platform 112, a suture nest 114, a pin retaining member 116, a pin 116a extending from pin retaining member 116, and a pin lock 118. Platform 112 includes one or more openings 112a for securing base 110 to a workstation (not shown) using bolts 112b or other fixation means. As shown, suture nest 114 is integrally formed with platform 112. Alternatively, suture nest 114 may be releasably attached or securely affixed to platform 112. Nest 114 includes one or more channels 115 extending across a top surface 114a thereof. As will be described in further detail below, channels 115 are configured to partially receive a portion of suture thread 11, including second section 14 (FIG. 1). Nest 114 may further includes raised outer portions 113 extending along proximal and distal ends 115a, 115b of channels 115. Raised outer portions 113 include openings 113a configured to receive two lengths of suture thread 11, adjacent to or on top of one another. As shown, suture nest 114 includes three channels 115; however, it is envisioned that suture nest 115 may include one or more channels 115. In one embodiment, suture nest 115 may be formed without a channel. In this manner, the first and second portions of suture thread 11 would be received in opening 113a and are maintained adjacent to one another through the tension applied by suture tensioning means 125 (FIG. 3A).

Still referring to FIGS. 2A-2C, pin 116a extends from pin retaining member 116. Pin retaining member 116 is pivotally attached to platform 112 such that pin 116a may be selectively positioned and securely retained perpendicular to channels 115 along an end thereof (FIG. 3B). Pin lock 118 is pivotally attached to suture nest 114 and is configured to secure pin 116a in the perpendicular position adjacent proximal end 115a of channels 115. Alternatively, pin lock 118 may be integrally formed with suture nest 114. In another alternate embodiment, pin retaining member 116 may be releasably attached or securely affixed to platform 112. The diameter of pin 116a may be varied depending on the desired size of loop 12.

Turning briefly to FIG. 3A, as discussed above, system 100 also includes a suture retaining means 120 and a suture tensioning means 125. Suture retaining means 120 may include a clamp or other device configured to retain a proximal end 11a of suture thread 11. Suture tensioning means 125 may include a hydraulic or pneumatic tensioning spring, electrical cylinder or other tensioning device configured to receive a distal end 11b of suture thread 11 and to apply tension to suture thread 11 once thread 11 has been secured to suture nest 114. Suture retaining means 120 and suture tensioning means 125 are positioned adjacent distal end 115b of channel 115 to securely receive respective proximal and distal ends 11a, 11b of suture thread 11 during the forming of loop 12.

With reference to FIGS. 3A-4B, welding assembly 130 includes an ultrasonic device 132 operably connected to a generator (not shown) for ultrasonically vibrating a die 134 extending from ultrasonic device 132. Die 134 defines a substantially flat suture contacting portion 136. In an alternative embodiment, die 134 may include a portion 136 configured to contour first section 13 of suture thread 11. Thus, suture contacting portion 136 may include a concave, convex or beveled surface to correspond with a suture thread having a convex, concave or beveled profile. In one embodiment, welding assembly 130 is operatively mounted on a press assembly (not shown) for approximating die 134 of welding assembly 130 towards and away from base 110. Alternatively, welding assembly 130 may be securely mounted relative to base 110 and base 110 may be raised and lowered to approximate base 110 towards and away from die 134.

Turning now to FIGS. 5A- 6B, cutting assembly 140 includes an ultrasonic device 142 operably connected to a generator (not shown) for ultrasonically vibrating a blade 144 extending from ultrasonic device 142. The generator for ultrasonically vibrating die 134 may be the same or a different generator as is operatively connected to ultrasonic device 142 for ultrasonically vibrating blade 144. In one embodiment, blade 144 defines a substantially flat cutting surface 144a; however, it is envisioned that blade 144 may include a cutting surface of alternative configurations. Blade 144 may be configured to form a concave, convex, beveled or otherwise configured taper 17 on looped suture 10. Although the following discussion will relate to a cutting assembly that includes an ultrasonic device 142, it is envisioned that cutting assembly 140 may be used without ultrasonically vibrating blade 144. In this manner, blade 144 may be operably connected to a heater or other apparatus for effecting cutting of suture thread 11. In yet another embodiment, taper 17 on looped suture 10 may be cut using a laser.

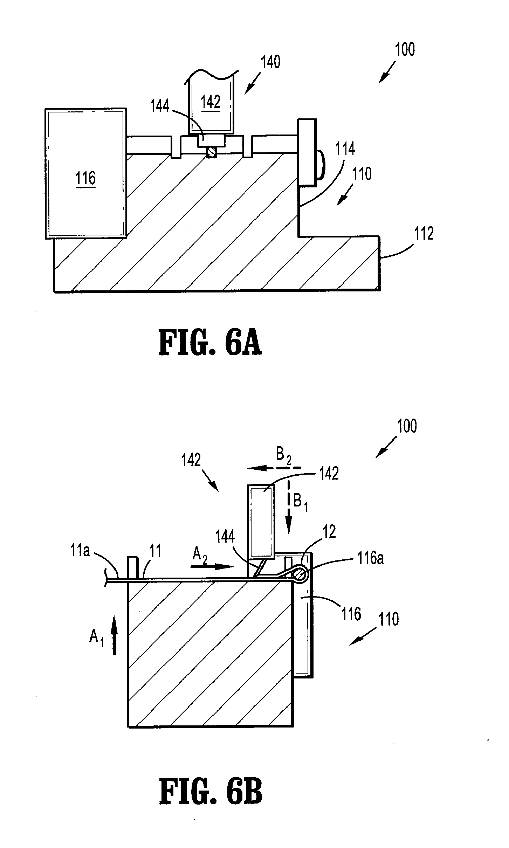

Still referring to FIGS. 5A-6B, in one embodiment, cutting assembly 140 is securely mounted relative to base 144 such that cutting assembly 140 is maintained stationary as base 110 is approximated towards and away from blade 144. In an alternative embodiment, cutting assembly 140 is selectively positioned relative to base 110, such that cutting assembly 140 moved relative to base 110. In either embodiment, at least one of cutting assembly 140 and base 110 is configured to move laterally with respect to the other and approximate towards the other. When cutting assembly 140 is maintained stationary, base 110 is configured to move laterally, in the direction of arrow A1 (FIG. 6B) and towards cutting assembly 140, in the direction of arrow A2. When base 110 is maintained stationary, cutting assembly 140 is configured to move laterally, in the direction of arrow B1 and towards base 110, in the direction of arrow A2. Movement of base 110 and/or cutting assembly 140 may be computer controlled or may be effected manually.

The method of forming looped suture 10 utilizing system 100 will now be described with reference to FIGS. 3A-6B. Referring initially to FIG. 3A, a proximal end 11a of thread 11 is securely locked in a clamp 120. Second section 14 of thread 11 is then positioned within a channel 115 of nest 114. Thread 11 is next wrapped around pin 116a before first section 13 of thread 11 is placed on top of or adjacent second section 14. A distal end 11b of thread 11 is then received in tension cylinder 125. Tensioning cylinder 125 is then activated to tension thread 11 within base 110. To prevent stretching of thread 11 during forming of looped suture 10, and thereby ensuring consistency and integrity of thread 11, thread 11 may be formed of a prestretched material.

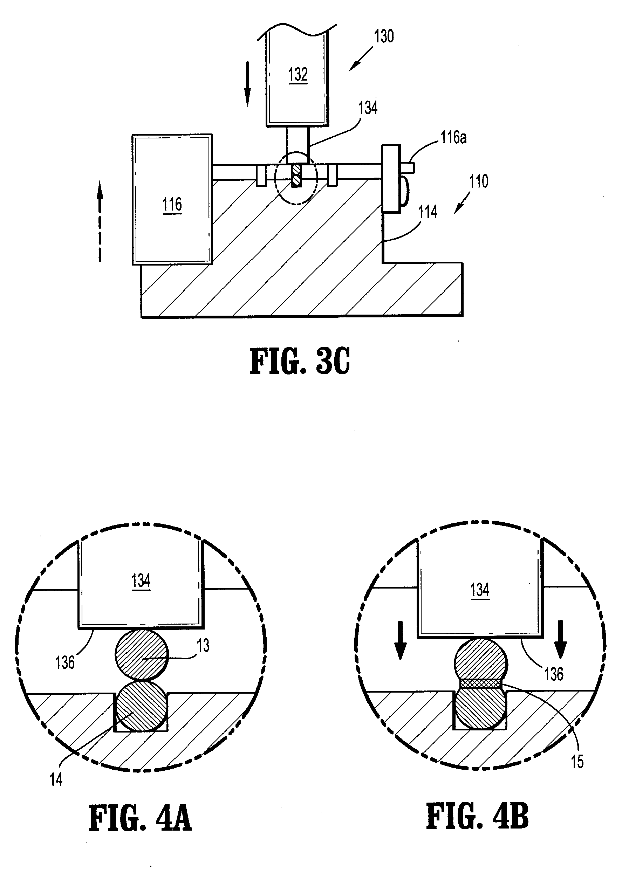

With particular reference now to FIGS. 3C-4B, once first and second sections 13, 14 are positioned adjacent one another, welding assembly 130 is approximated towards suture nest 114. Alternatively, suture nest 114 may be approximated towards welding assembly 130. As welding assembly 130 nears suture nest 114, first section 13 of suture thread 11 is received within channel 136 of die 134 until first section 13 engages suture contacting portion 136a of channel 136 (FIG. 4A). Ultrasonic device 132 may be activated at any point during this process to ultrasonically vibrate die 134. The downward pressure exerted on first and second sections 13, 14 of thread 11 from the continued approximation of die 134 towards nest 114 (FIG. 4B), in combination with ultrasonic vibration of die 134, causes contacting portions of first and second sections 13, 14 to locally heat, and, in some instances, the contacting portions may begin to melt. Application of ultrasonic energy to sections 13, 14 creates joined section 15.

Once first and second sections 13, 14 are fused to create joined section 15, welding assembly 130 may be approximated away from suture nest 114. With looped suture 10 remaining secured to base 110, welding assembly 130 may then be replaced or exchanged for cutting assembly 140 to complete the tapered cutting of proximal end 13a of first section 13. Alternatively, looped suture 10 may be removed from base 110 and affixed to a separate mount (not shown) to complete the taper forming process.

With reference now to FIGS. 5A-6B, once cutting assembly 130 is properly positioned in relation to base 110, base 110 is approximated towards cutting assembly 140. Alternatively, cutting assembly 140 may be approximated towards base 110. Ultrasonic device 142 may be activated at any time prior to or during approximation of cutting assembly 140 towards base 110. With particular reference to FIG. 6B, the travel of base 110 in relation to cutting assembly 140 includes an upward movement, as indicated by arrow A₁ and lateral movement, as indicated by arrow A₂. In an alternative embodiment, cutting assembly 140 is moved in a downward movement, as indicated by arrow B₁ and a lateral movement, as indicated by arrow B₂. The rate at which base 110 moves relative to cutting assembly 140 may be varied depending on the desired configuration of tapered surface 17 (FIG. 1C). In this manner, when the lateral movement is increased relative to the up/down movement, tapered surface 17 defines an angle α (FIG. 1C) of a lesser degree. Conversely, when the lateral movement of is decreased relative to the up/down movement, angle α of tapered surface 17 is increased. In either embodiment, cutting assembly 130 and base 110 are approximated toward one another until blade 144 completely severs first section 13 of thread 11. Distal end 11b of thread 11 is then pulled away by tension cylinder 125.

Cutting assembly 130 and base 110 are then approximated away from each other and looped suture 10 is removed from pin 116a. Suture 10 may include flash or debris (not shown) formed during the welding and/or cutting process. The flash may need to be removed before looped suture 10 may be used.

Although the illustrative embodiments of the present disclosure have been described herein with reference to the accompanying drawings, it is to be understood that the disclosure is not limited to those precise embodiments, and that various other changes and modifications may be effected therein by one skilled in the art without departing from the scope or spirit of the disclosure.

The invention may be described by reference to the following numbered paragraphs:-
1. A method of forming a loop in a thread, the method comprising the step of:
   providing a loop forming system including, a base for securely retaining a thread to be formed and a welding assembly for forming a loop in the thread;
   providing a chamber filled with super critical fluid;
   securing the thread to the base such that a first section of the thread is maintained adjacent to a second section of the thread;
   positioning the base in the chamber;
   approximating the welding assembly and the base towards each other to ultrasonically weld the first and second section of thread together; and
   approximating the welding assembly and base away from each other.
2. The method of paragraph 1, wherein the super critical fluid is super-critical carbon dioxide (CO₂).
3. A medical device formed from the method paragraph 1.

## Claims

1. A method of forming a loop in a thread, the method comprising the step of:
providing a loop forming system including, a base for securely retaining a thread to be formed and a welding assembly for forming a loop in the thread;
contacting at least a portion of the thread with a super-critical fluid;
securing the previously immersed portion of the thread to the base such that a first section of the previously immersed portion is maintained adjacent to a second section of the previously immersed portion;
approximating the welding assembly and the base towards each other; and
approximating the welding assembly and base away from each other.

2. The method of claim 1, wherein the loop forming system further includes a cutting assembly for forming a taper on an end of the loop.

3. The method of claim 2, further including the steps of:
approximating the cutting assembly and the base towards each other;
cutting a tapered end on a proximal end of the first section of the thread; and
approximating the cutting assembly and base away from each other.

4. The method of any preceding claim, further including the step of removing the formed suture from the base.

5. The method of any preceding claim, wherein the loop forming system further includes a clamping device for receiving the first end of a thread.

6. The method of any preceding claim, wherein the loop forming system further includes a tensioning device for tensioning the thread once the thread is retained in the base.

7. The method of any preceding claim, wherein the step of contacting at least a portion of the thread with a super critical fluid includes submersing the at least a portion of the thread in the super critical fluid.

8. The method of any one of claims 1 to 6, wherein the step of contacting at least a portion of the thread with a super critical fluid includes applying the super critical fluid to the at least a portion of the thread.

9. A medical device formed by the method of any preceding claim.

10. A method of forming a loop in a suture, the method comprising the steps of:
providing a length of thread having a first section and a second section;
contacting at least the first section and the second section of the thread with a super-critical fluid;
overlapping the first and second sections of thread; and
pressing together the first and second sections of thread.

11. The method of claim 10, wherein the step of contacting the at least first and second sections of thread with a super critical fluid includes submersing the at least first and second sections of thread in the super critical fluid.

12. The method of claim 10, wherein the step of contacting the at least first and second sections of thread with a super critical fluid includes applying the super critical fluid to the at least first and second sections of thread.

13. The method of any one of claims 10 to 12, further including the step of cutting a tapered end on the proximal end of the first section of thread.

14. The method of any preceding claim, wherein the super critical fluid is super-critical carbon dioxide (CO₂).

15. A medical device formed by the method of any one of claims 10 to 14.
